# EUROPÄISCHE PATENTANMELDUNG

(11) **EP 3 611 284 A2**
(43) Veröffentlichungstag der Anmeldung: **19.02.2020**
(21) Anmeldenummer: 19189426.0
(22) Anmeldetag: 31.07.2019
(51) Int. Cl.: C22C 23/06, C22F 1/06, A61L 27/04, A61L 31/02

(54) **BIODEGRADIERBARES DRAHTIMPLANTAT**

(30) Priorität: 17.08.2018 DE 102018120093
(71) Anmelder: Syntellix AG, 30159 Hannover (DE)
(72) Erfinder: Jan Marten, Seitz, 30159 Hannover (DE); Katrin, Nagel, 30519 Hannover (DE)
(74) Vertreter: Dennemeyer & Associates S.A.

(57) **Zusammenfassung**

Die Erfindung betrifft ein Drahtimplantat, insbesondere zur Draht-Osteosynthese, sowie ein entsprechendes Verfahren zu dessen Herstellung. Vorgesehen ist, dass das Drahtimplantat einer Wärmebehandlung unterzogen wurde, wobei das Drahtimplantat aus einer biokompatiblen, biokorrodierbaren Magnesiumlegierung besteht, die zusammengesetzt ist aus metallischem Magnesium von mindestens 80 Gew. %, einem Anteil von Zink von 0,1 bis 2,0 Gew. %, einem Anteil von Zirkonium von 0,1 bis 2,0 Gew. %, einem Anteil von Selten-Erd-Metallen von 0,1 bis 10 Gew. % - wobei der Yttrium Gehalt unter dem Selten-Erd-Metall-Anteil 0,1 bis 5,0 Gew. % beträgt, einem Anteil von Mangan von 0,01 bis 0,2 Gew. %, einem Anteil von Aluminium von weniger als 0,1 Gew. %, einem Anteil von Kupfer, Nickel und Eisen von jeweils weniger als 0,10 Gew. %, und einem Anteil sonstiger physiologisch unerwünschte Verunreinigungen von insgesamt weniger als 0,8 Gew. %, enthält wobei der Rest der Legierung zu 100 Gew. % Magnesium ist.

## Beschreibung

Die Erfindung betrifft ein Drahtimplantat, insbesondere zur Draht-Osteosynthese, sowie ein entsprechendes Verfahren zu dessen Herstellung.

### Stand der Technik

Bei einem Knochenbruch ist oftmals ein Osteosyntheseverfahren notwendig, um die zueinander gehörigen Knochenfragmente in einer anatomisch korrekten Stellung zu verbinden und zu fixieren. Je nach Art des Bruches werden unterschiedliche Fixationsmittel und Verfahren angewendet.

Bei der Draht-Osteosynthese werden Drahtimplantate verwendet, um die Knochenfragmente zu verbinden und zu fixieren.

Bei der Spickdrahtosteosynthese werden die Knochenbruchstücke nach Reposition mittels sogenannter Spick- oder Kirschner Drähte (K-Draht) fixiert. Diese Bohrdrähte bestehen zumeist aus Edelstahl oder Titan und werden durch Rotation in den Knochen eingebohrt. Die Kirschner-Draht-Osteosynthese geht auf ihren Erfinder und Entwickler Dr. Martin Kirschner (1879 - 1942) zurück. Der Vorteil einer Osteosynthese mit Drähten liegt darin, dass sie über relativ kleine Hautschnitte erfolgen kann. Nach Abschluss der Knochenbruchheilung müssen die Drähte wieder entfernt werden.

Drahtimplantate können auch verwendet werden, um Knochenfragmente zu umschlingen und auf diese Weise zu verbinden und zu fixieren (Drahtcerclage).

Das Entfernen der Drähte geschieht zumeist unter Narkose, was immer ein gewisses Narkoserisiko mit sich bringt. Zudem bleiben nach dem Entfernen von K-Drähten aus Edelstahl oder Titan immer und unvermeidbar kleine Öffnungen und Hohlräume im Knochen zurück, die erneut abheilen müssen.

Um die Nachteile der Implantat-Entfernung zu vermeiden, werden in jüngster Zeit immer häufiger bio-resorbierbare Implantate zur Osteosynthese eingesetzt. Sie müssen nach der Heilung nicht operativ entfernt werden, da sie sich im Körper auflösen und teilweise sogar in Knochensubstanz umgewandelt werden.

Die WO 2015/137911 A1 beschreibt eine orthopädische Befestigungsvorrichtung, welche als K-Draht gestaltet sein kann und mit eineKdoEnu12m abbaubaren Hydrogel beschichtet ist. Nach Implantation der Befestigungsvorrichtung nimmt das Hydrogel an Volumen zu und trägt so zur inneren Fixierung der Fraktur bei. Das Hydrogel wird mit der Zeit abgebaut. Das Implantat kann aus Magnesium aufgebaut sein.

In der WO 2017/035072 A1 werden abbaubare, auf Magnesium basierende Implantate zur Knochen Fixierung beschrieben. Die Magnesiumlegierung kann dabei auch als Draht verwendet werden, der zur Fixierung um Knochen herum gewickelt wird (Drahtcerclage), oder als Pin.

### Aufgabe

Aufgabe der Erfindung ist es, ein degradierbares Drahimplantat bereit zu stellen, welches eine hohe Festigkeit bei gleichzeitiger hoher Elastizität bietet.

Für biodegradierbare Drahtimplantate sind einige besondere mechanische Eigenschaften des resorbierbaren Metalls erforderlich, die für andere Implantate wie Schrauben, Nägel, Platten oder Schienen von untergeordneter Rolle sein können. So ist insbesondere eine hohe Steifigkeit bei gleichzeitiger hoher Festigkeit des Drahtes notwendig. Andernfalls würde sich der K-Draht beim Eindrehen in den Knochen verwinden, in sich verdrehen oder abknicken.

### Lösung

Diese Aufgabe wird durch die Gegenstände der unabhängigen Ansprüche gelöst. Vorteilhafte Weiterbildungen der Gegenstände der unabhängigen Ansprüche sind in den Unteransprüchen gekennzeichnet. Der Wortlaut sämtlicher Ansprüche wird hiermit durch Bezugnahme zum Inhalt dieser Beschreibung gemacht. Die Verwendung der Einzahl soll die Mehrzahl nicht ausschließen, was auch im umgekehrten Sinn zu gelten hat, soweit nichts Gegenteiliges offenbart ist.

Zur Lösung der Aufgabe wird ein Drahtimplantat, insbesondere zur Draht-Osteosynthese vorgeschlagen, zur Positionierung und/oder Fixierung mindestens eines gebrochenen oder osteotomierten Knochens, wobei
das Drahtimplantat einer Wärmebehandlung unterzogen wurde,
und wobei das Drahtimplantat aus einer biokompatiblen, biokorrodierbaren Magnesiumlegierung besteht, die zusammengesetzt ist aus metallischem Magnesium von mindestens 80 Gew. %, einem Anteil von Zink von 0,1 bis 2,0 Gew. %, einem Anteil von Zirkonium von 0,1 bis 2,0 Gew. %, einem Anteil von Selten-Erd-Metallen von 0,1 bis 10 Gew. % - wobei der Yttrium Gehalt unter dem Selten-Erd-Metall-Anteil 0,1 bis 5,0 Gew. % beträgt, einem Anteil von Mangan von 0,01 bis 0,2 Gew. %, einem Anteil von Aluminium von weniger als 0,1 Gew. %, einem Anteil von Kupfer, Nickel und Eisen von jeweils weniger als 0,1 Gew. %, und einem Anteil sonstiger physiologisch unerwünschter Verunreinigungen von insgesamt weniger als 0,8 Gew. %, enthält wobei der Rest der Legierung zu 100 Gew. % Magnesium ist.

Der Begriff Drahtimplantat umfasst dabei sämtliche Drähte, wie Spick- oder Kirschner Drähte oder auch Drahtcerclagen, die zur Osteosynthese verwendet werden können.

Vorzugsweise enthält die Magnesiumbasislegierung Magnesium von mindestens 88 Gew. %, 0,10 bis 1,00 Gew.-% Zirkonium, 0,01 bis 1,00 Gew.-% Zink, 1,00 bis 3,00 Gew.-% Yttrium und 2,00 bis 5,00 Gew.-% sonstige Seltenerdmetalle. Es ist dabei bevorzugt, dass die Magnesiumbasislegierung bezogen auf die Legierung insgesamt einen Gehalt von physiologisch unerwünschten Verunreinigungen der Metalle Eisen, Kupfer, Nickel und Aluminium von jeweils weniger als 0,02 Gew.-% aufweist. Insbesondere enthält die Magnesiumbasislegierung unter 0,01 Gew.-% Aluminium, unter 0,20 Gew.-% Eisen, unter 0,20 Gew.-% Mangan und jeweils weniger als 0,02 Gew.-% Kupfer und Nickel. Weiterhin ist es bevorzugt, dass die Magnesiumbasislegierung unter 0,01 Gew.- % Aluminium, unter 0,20 Gew.-% Zink, unter 0,15 Gew.-% Mangan, unter 0,20 Gew.-% Lithium, unter 0,01 Gew.-% Silizium, unter 0,01 Gew.-% Eisen, unter 0,03 Gew.-% Kupfer und unter 0,005 Gew.-% Nickel enthält.

Unter der Bezeichnung "unvermeidbare sonstige Verunreinigungen" sind Elemente zu verstehen, die nach allgemeiner medizinischer Erfahrung keine negativen physiologischen Wirkungen erwarten lassen und aufgrund der niemals 100 %-igen Reinheit der Einsatzstoffe nicht völlig ausgeschlossen werden können. Beispiele sind Kohlenstoff, Silizium, Natrium, Kalium, Sauerstoff, Stickstoff, Wasserstoff, Calcium.

Erfindungsgemäß müssen die Drähte aber zusätzlich einer Wärmebehandlung unterzogen werden, um die ausreichende Torsionsfestigkeit für lange und dünne stricknadelförmige Implantate zu erreichen.

In einer Ausführungsform wird das Drahtimplantat einer Wärmebehandlung durch Lösungsglühen, Abschrecken und anschließendem Warmauslagern unterzogen. Ebenso ist ein Weichglühen möglich. Diese Aufzählung ist nicht abschließend. Es sind auch weitere Methoden der Wärmebehandlung für den Fachmann möglich.

Die durch Wärmebehandlungen behandelte Drähte oder Drahtabschnitte sind form- und biegbar. Zudem kann durch die Wärmebehandlung auch das Degradationsverhalten des Drahtes in einzelnen Abschnitten unterschiedlich geprägt sein. Auf diese Weise ist es möglich, ein auf den Patienten bzw. die notwenige Behandlung individuell angepasstes Drahtimplantat zur Verfügung zu stellen.

Die grundlegenden mechanischen Eigenschaften des Drahtimplantats werden demnach durch die Zusammensetzung der Magnesiumlegierung, sowie die nachfolgende Wärmebehandlung bzw. durch das Zusammenwirken dieser Faktoren maßgeblich bestimmt.

Durch die Wärmebehandlung können einerseits im wärmebehandelten Bereich die Werte der Dehngrenze bzw. Zugfestigkeit signifikant erniedrigt werden und die Bruchdehnung steigt an. Die durch Wärmebehandlungen behandelten Drähte oder Drahtabschnitte sind somit form- und biegbar. Andererseits können durch die Wärmebehandlung auch besonders feste Bereiche, in Bezug auf Torsion und Biegung, erzeugt werden.

Durch das Weichglühen werden die Werte der Dehngrenze signifikant erniedrigt und die Bruchdehnung steigt signifikant an. Dies führt in der Praxis zu einer guten Form- und Biegbarbarkeit des Drahts an den so behandelten Stellen.

Durch das Auslagern werden insbesondere hohe Festigkeiten erzielt, welche insbesondere die Zug- und Torsionsfestigkeit des Drahtes an den so behandelten Stellen verbessern.

Drähte mit Segmenten unterschiedlicher Festigkeit bzw. Duktilität haben den Vorteil, dass beispielsweise tribologisch beanspruchte Teile eines Implantates, z.B. die für das Bohren vorgesehene Spitze, besonders fest und damit hart ausgelegt werden können und so die auftretende Schneidarbeit und der Verschließ/Abrieb bei der Implantation gering gehalten werden. Gleichzeitig gewährt der nachfolgende duktilere Anteil des Drahtes eine Anformung an das Operationsgebiet und ermöglicht sogar die Ausbildung einer Cerclage.

Weiter können die Segmente des Drahtes, resultierend aus den unterschiedlichen Wärmebehandlungen, unterschiedliche Degradationsraten aufweisen, mit welchen beispielsweise die Einflüsse von Arealen unterschiedlicher Gewebe auf das Degradationsverhalten der verwendeten Magnesiumlegierung gezielt ausgeglichen werden können. So ist das Implantat beispielsweise so gestaltbar, dass der im Weichteil verbleibende Drahtanteil eine deutlich geringere Degradationsrate aufweist, als der im Knochen befindliche. Da die Degradation von Magnesium im Weichgewebe aufgrund des höheren Wasseranteils und der höheren Stofftransportrate rascher abläuft, wäre für das hier eingesetzte Drahtsegment eine Wärmebehandlung, welche in einer geringen Degradationsrate resultiert vorteilhaft. Grundsätzlich kann auf diese Weise der Degradationsfortschritt auch so gesteuert werden, dass sich der Draht gerichtet von seinen Enden Auflöst und nicht gleichmäßig über seine gesamte Oberfläche. Dies kann den Vorteil mit sich bringen, dass das Implantat an kritischen Stellen besonders lange intakt bleibt.

Zudem kann durch die Wärmebehandlungen das Degradationsverhalten des Drahtes in einzelnen Abschnitten unterschiedlich geprägt sein. Durch die Wärmebehandlung sind in dem jeweiligen wärmebehandelten Bereich Degradationsraten von 0,05 bis 2,0 mm pro Jahr möglich.

Auf diese Weise ist es möglich, ein auf den Patienten bzw. die notwenige Behandlung individuell angepasstes Drahtimplantat zur Verfügung zu stellen.

In einer Ausführungsform wird das Drahtimplantat über die gesamte Länge wärmebehandelt. Es kann aber erwünscht sein, in nur einem Teilabschnitt oder auch mehreren Teilabschnitten des Drahtes eine gewisse Verformbarkeit und Biegsamkeit herzustellen, um verschobene, aufgereihte Knochenfragmente in die anatomisch korrekte Position drücken zu können. In einer weiteren Ausführungsform erfolgt die Wärmebehandlung in mindestens einem Teilabschnitt. Insbesondere ist eine Wärmebehandlung etwa im mittleren Drittel von Vorteil, vorzugsweise in einem Drittel bis zu zwei Drittel der Gesamtlänge des Drahtimplantats.

Die unterschiedlichen Möglichkeiten der Wärmebehandlung wie Auslagern und Weichglühen können auch an einem Draht angewendet werden. So kann beispielsweise ein Draht mit einer Spitze mit hoher Festigkeit erzeugt werden und der übrige Teil des Drahtes ist weicher uns biegsamer.

Die Wärmebehandlung kann über die gesamte Länge des Drahtimplantats oder in mindestens einem Teilabschnitt erfolgen. In einer Ausführungsform wird das degradierbare Drahtimplantat über die gesamte Länge wärmebehandelt. Es kann aber erwünscht sein, in nur einem Teilabschnitt oder auch mehreren Teilabschnitten des Drahtes eine gewisse Verformbarkeit und Biegsamkeit herzustellen, um verschobene, aufgereihte Knochenfragmente in die anatomisch korrekte Position drücken zu können. Insbesondere ist eine Wärmebehandlung etwa im mittleren Drittel von Vorteil. Der Begriff Teilabschnitt im Sinne dieser Erfindung umfasst dabei mindestens einen Bereich von 1 % der Gesamtlänge des fertigen Implantats.

Weiterhin ist es vorgesehen, dass die Bruchdehnung in einem weichgeglühten Bereich mindestens 18% beträgt, vorzugsweise mindestens 20%. Auch ist vorgesehen, dass die Bruchdehnung in einem dem Auslagern unterzogenen Bereich maximal 3,5 % beträgt.

In einer weiteren Ausgestaltungsform beträgt die Dehngrenze in einem dem Auslagern unterzogenen Bereich mindestens 360 MPa beträgt, vorzugsweise mindestens 380 MPa. Zudem ist es vorteilhaft, wenn die Dehngrenze in einem weichgeglühten Bereich mindestens 240 MPa beträgt.

Günstig ist es, wenn die Zugfestigkeit in einem weichgeglühten Bereich mindestens 390 MPa beträgt.

Ebenfalls ist es vorgesehen, dass der Durchmesser des Drahtimplantats 0,2 mm bis 6,0 mm beträgt, vorzugsweise 0,5 mm bis 4,0 mm und die Länge 30 mm bis 600 mm des Drahtimplantats beträgt, vorzugsweise 50 mm bis 500 mm.

Der endgültige Durchmesser, die endgültige Gestalt und Form der degradierbaren Drahtimplantate wird vorzugsweise aus stangenförmigen Halbzeugen durch Kalt- oder Warmumformung, d.h. durch die allgemein bekannten Methoden des Walzens, Strangpressens und Drahtziehens hergestellt. Diese Aufzählung ist nicht abschließend. Es sind auch weitere Methoden für den Fachmann denkbar.

Günstig ist es, wenn der Draht rund oder mehrkantig oder mit Längsrillen gestaltet ist. Auch sind gewindeartige Vertiefungen möglich. Anstelle runder Drähte können auch mehrkantige Drähte, beispielsweise drei-kant oder vier-kant, oder kannülierte Drähte mit Längsrillen erzeugt werden. Diese können durch Strangpressen oder durch Ziehen mittels geeigneter Matrizen erzeugt werden. Auch ist es denkbar, dass die Form des Drahtes nach seiner Herstellung mittels geeigneter Verfahren verändert wird, beispielsweise mittels fräsen.

Die Enden des degradierbaren Drahtimplantats, können flach oder mit einer Spitze gestaltet sein. Es ist vorteilhaft, dass das degradierbare Drahtimplantat, insbesondere eines K-Drahts, an mindestens einem der Enden eine Spitze aufweist. Die Spitze kann beispielsweise als Trokar-, Lanzett-, Meißel-Spitze oder auch als Bohreransatz gestaltet sein. Die gängigen Trokar- oder lanzenförmigen Spitzen können durch Fräsen, Schleifen, spanabhebendes Drehen oder Formpressen angebracht werden. Auch ist es vorgesehen, dass mindestens an einem der Enden des degradierbaren Drahtimplantats, insbesondere eines K-Drahts, ein ösenförmiges Loch vorhanden ist.

In einer weiteren Ausgestaltungsform weist das Drahtimplantat in mindestens einem Teilabschnitt eine glatte Oberfläche auf, vorzugsweise eine glatt polierte Oberfläche.

Günstig ist es, wenn das Drahtimplantat in mindestens einem Teilabschnitt eine Rautiefe von weniger als 1,0 µm aufweist, vorzugsweise von 0,8 µm. Die Rauigkeit wird dabei gemessen als Rz Wert (gemittelte Rautiefe). Die Rautiefe kann beispielsweise durch mechanisches Polieren, durch oszillierendes Kreuzschleifen, so genanntes Superfinishen, erzielt werden. Auch durch spezielle Methoden des elektrochemischen Polierens kann man diese geringe Rauigkeit erzielen. Durch die geringe Rauigkeit wird eine Verlängerung der Degradationszeit bzw. eine Verminderung der Degradationsrate erzielt.

Es wurde dabei festgestellt, dass eine Rautiefe von weniger als 1,0 µm zu einer Verzögerung der Degradation um 100 - 200 Stunden führt.

Weiterhin ist es vorgesehen, dass das Drahtimplantat in mindestens einem Teilabschnitt hohl gestaltet ist. In den Hohlraum können vorzugsweise ein oder mehrere Substanzen, vorzugsweise Medikamente, eingebettet sein, wodurch die Möglichkeit der postoperativen medikamentösen Behandlung des Patienten gegeben ist. Im Zuge der Degradation des Implantats wird dieses integrierte Medikamentendepot mit der Zeit zugänglich und eine Abgabe des Medikaments ans umliegende körpereigene Knochen- und Weichgewebe wird ermöglicht. Auch ist es möglich, eine Substanz in dem Hohlraum einzusetzen, anhand dessen Freisetzung die Degradationsrate bestimmt werden kann.

Die Aufgabe wird ferner gelöst durch ein Verfahren zur Herstellung eines Drahtimplantats,
wobei das Drahtimplantat über die gesamte Länge des Drahtes oder in mindestens einem Teilabschnitt des Drahtimplantates einer Wärmebehandlung unterzogen wird,
und wobei das Drahtimplantat aus einer biokompatiblen, biokorrodierbaren Magnesiumlegierung besteht, die zusammengesetzt ist aus metallischem Magnesium von mindestens 80 Gew. %, einem Anteil von Zink von 0,1 bis 2,0 Gew. %, einem Anteil von Zirkonium von 0,1 bis 2,0 Gew. %, einem Anteil von Selten-Erd-Metallen von 0,1 bis 10 Gew. % - wobei der Yttrium Gehalt unter dem Selten-Erd-Metall-Anteil 0,1 bis 5,0 Gew. % beträgt, einem Anteil von Mangan von 0,01 bis 0,2 Gew. %, einem Anteil von Aluminium von weniger als 0,1 Gew. %, einem Anteil von Kupfer, Nickel und Eisen von jeweils weniger als 0,10 Gew. %, und einem Anteil sonstiger physiologisch unerwünschte Verunreinigungen von insgesamt weniger als 0,8 Gew. %, enthält wobei der Rest der Legierung zu 100 Gew. % Magnesium ist.

Zur Herstellung des biodegradierbaren Drahtimplantats wird vorzugsweise zunächst eine Magnesiumlegierung erzeugt, die vorteilhafterweise zusammengesetzt ist aus metallischem Magnesium von mindestens 80 Gew. %, einem Anteil von Zink von 0,1 bis 2,0 Gew. %, einem Anteil von Zirkonium von 0,1 bis 2,0 Gew. %, einem Anteil von Selten-Erd-Metallen von 0,1 bis 10 Gew. % - wobei der Yttrium Gehalt unter dem Selten-Erd-Metall-Anteil 0,1 bis 5,0 Gew. % beträgt, einem Anteil von Mangan von 0,01 bis 0,2 Gew. %, einem Anteil von Aluminium von weniger als 0,1 Gew. %, einem Anteil von Kupfer, Nickel und Eisen von jeweils weniger als 0,1 Gew. %, und einem Anteil sonstiger physiologisch unerwünschte Verunreinigungen von insgesamt weniger als 0,8 Gew. %, enthält wobei der Rest der Legierung zu 100 Gew. % Magnesium ist.

Mittels geeigneter gängiger Verfahren der Kalt- oder Warmumformung, wie dem Strangpressen, einem Verfahren des Kalt- und Heiß-Pressens oder des Kompaktierens, des Schmiedens oder des Walzens oder weitere Verfahren der Kalt- oder Warmumformung wird vorzugsweise ein Magnesiumformteil gefertigt.

Ein auf diese Weise gefertigtes Formteil wird anschließend zum gewünschten Drahtimplantat weiterverarbeitet. Der endgültige Durchmesser, die endgültige Gestalt und Form der Drahtimplantate wird aus Halbzeugen vorzugsweise durch Kalt- oder Warmumformung, insbesondere durch die allgemein bekannten Methoden des Walzens, Strangpressens und Drahtziehens hergestellt. Diese Aufzählung ist nicht abschließend. Es sind auch weitere Methoden für den Fachmann denkbar.

Die Wärmebehandlung des Drahtimplantates erfolgt über die gesamte Länge des Drahtes oder in mindestens einem Teilabschnitt.

Unter Wärmebehandlung im Sinne dieser Erfindung ist dabei eine Behandlung des Drahtimplantates in einem oder mehreren Schritten zu verstehen, wobei das Drahtimplantat in einem bestimmten Verlauf erwärmt und wieder abgekühlt wird, um Werkstoffeigenschaften zu verändern.

Durch die Wärmebehandlung, insbesondere durch Lösungsglühen, Abschrecken und anschließendes Warmauslagern oder auch mittels Weichglühen, erhalten die Drähte die ausreichende Torsionsfestigkeit sowie Biegefestigkeit für lange, dünne und stricknadelförmige Bauteile.

In einer Ausführungsform wird das Drahtimplantat einer Wärmebehandlung durch Lösungsglühen, Abschrecken und anschließenden Warmauslagern unterzogen. Ebenso ist ein Weichglühen möglich. Diese Aufzählung ist nicht abschließend. Es sind auch weitere Methoden der Wärmebehandlung für den Fachmann möglich.

Vorzugsweise erfolgt das Lösungsglühen bei einer Temperatur von 300°C bis 550°C, vorzugsweise von 350°C bis 500°C, besonders bevorzugt bei 480°C. Das Lösungsglühen erfolgt dabei vorzugsweise über einen Zeitraum von 2 bis 100 Minuten, besonders bevorzugt 3 bis 60 Minuten. Das Abschrecken erfolgt vorzugsweise in Wasser, Öl oder mit Kaltluft. Das Warmauslagern erfolgt vorzugsweise bei 120 bis 250°C, besonders bevorzugt bei 180°C über einen Zeitraum von 2h - 48h.

Es ist dabei vorgesehen, dass das Lösungsglühen berührungslos durch Laserlicht oder durch induktives Erwärmen oder durch Erwärmen mittels Infrarot-Bestrahlung oder in einem Ofen, beispielsweise einem Muffelofen oder einem Röhrenofen, vorzugsweise unter Schutzgas oder im (Teil)-Vakuum, ausgeführt wird.

In einer weiteren Ausführungsform wird das Drahtimplantat als Wärmebehandlung dem Weichglühen unterzogen. Vorzugsweise erfolgt das Weichglühen bei 350 bis 420°C, besonders bevorzugt bei 400°C, über einen Zeitraum von 5 bis 60 Minuten. Wird das gesamte Drahtimplantat weichgeglüht, so sinkt die Festigkeit, aber der Draht wird verformbar. Soll nur ein Abschnitt des Drahtimplantats weich werden, muss der Bereich, der zäh und steif bleiben soll, z.B. die Trokar-Spitze, während der Prozedur gekühlt werden bzw. darf nicht in der Einflusszone der Wärmebehandlung liegen.

Durch die Wärmebehandlung können einerseits im wärmebehandelten Bereich die Werte der Dehngrenze bzw. Zugfestigkeit signifikant erniedrigt werden und die Bruchdehnung steigt an. Die durch Wärmebehandlungen behandelten Drähte oder Drahtabschnitte sind somit form- und biegbar. Andererseits können durch die Wärmebehandlung auch besonders feste Bereiche, in Bezug auf Torsion und Biegung, erzeugt werden.

Durch das Weichglühen werden die Werte der Dehngrenze signifikant erniedrigt und die Bruchdehnung steigt signifikant an. Dies führt in der Praxis zu einer guten Form- und Biegbarbarkeit des Drahts an den so behandelten Stellen.

Durch das Auslagern werden insbesondere hohe Festigkeiten erzielt, welche insbesondere die Zug- und Torsionsfestigkeit des Drahtes an den so behandelten Stellen verbessern.

Zudem kann durch die Wärmebehandlungen das Degradationsverhalten des Drahtes in einzelnen Abschnitten unterschiedlich geprägt sein. Durch die Wärmebehandlung sind in dem jeweiligen wärmebehandelten Bereich Degradationsraten von 0,05 bis 2,0 mm pro Jahr möglich.

In einer Ausführungsform wird das Drahtimplantat über die gesamte Länge wärmebehandelt. Es kann aber erwünscht sein, in nur einem Teilabschnitt oder auch mehreren Teilabschnitten des Drahtes eine gewisse Verformbarkeit und Biegsamkeit herzustellen, um verschobene, aufgereihte Knochenfragmente in die anatomisch korrekte Position drücken zu können. In einer weiteren Ausführungsform erfolgt die Wärmebehandlung in mindestens einem Teilabschnitt. Insbesondere ist eine Wärmebehandlung etwa im mittleren Drittel von Vorteil, vorzugsweise in einem Drittel bis zu zwei Drittel der Gesamtlänge des Drahtimplantats.

Die unterschiedlichen Möglichkeiten der Wärmebehandlung wie Auslagern und Weichglühen können auch an einem Draht angewendet werden. So kann beispielsweise ein Draht mit einer Spitze mit hoher Festigkeit erzeugt werden und der übrige Teil des Drahtes ist weicher uns biegsamer.

Weiterhin ist es von Vorteil, wenn das Drahtimplantat in mindestens einem Teilabschnitt eine glatte Oberfläche, vorzugsweise eine glatt polierte Oberfläche, aufweist. Es festgestellt, dass sehr glatt polierte Oberflächen der degradierbaren Drahtimplantate zu der fast immer erwünschten Verzögerung des Degradationsbeginns führen. Die geringe Rautiefe kann über die gesamte Länge des Drahtes oder in mindestens einem Teilabschnitt erstrecken. Es wurde dabei festgestellt, dass eine Rautiefe von weniger als 1,0 µm zu einer Verzögerung der Degradation um 100 - 200 Stunden führt.

Günstig ist es, wenn das Drahtimplantat in mindestens einem Teilabschnitt eine Rautiefe von weniger als 1,0 µm aufweist, vorzugsweise von 0,8 µm. Die Rauigkeit wird dabei gemessen als Rz Wert (gemittelte Rautiefe). Die Rautiefe kann beispielsweise durch mechanisches Polieren, durch oszillierendes Kreuzschleifen, so genanntes Superfinishen, erzielt werden. Auch durch spezielle Methoden des elektrochemischen Polierens oder Läppen kann man diese geringe Rauigkeit erzielen. Durch die geringe Rauigkeit wird eine Verlängerung der Degradationszeit bzw. eine Verminderung der Degradationsrate erzielt.

Ein weiterer Gegenstand der vorliegenden Erfindung betrifft ein Drahtimplantat, welches nach dem vorgehend beschriebenen Verfahren erhältlich ist.

Weitere Einzelheiten und Merkmale ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Möglichkeiten, die Aufgabe zu lösen, sind nicht auf die Ausführungsbeispiele beschränkt. So umfassen beispielsweise Bereichsangaben stets alle - nicht genannten - Zwischenwerte und alle denkbaren Teilintervalle.

Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente. Im Einzelnen zeigt:
- Fig. 1: ein biodegradierbares Drahtimplantat;
- Fig. 2: ein biodegradierbares Drahtimplantat mit einem wärmebehandelten Abschnitt und mit einer Trokar-Spitze;
- Fig. 3: ein biodegradierbares Drahtimplantat mit polierten Abschnitten;
- Fig. 4: ein biodegradierbares Drahtimplantat mit 6 - kantigem Außenprofil; und
- Fig. 5: ein biodegradierbares Drahtimplantat mit kannüliertem Profil.

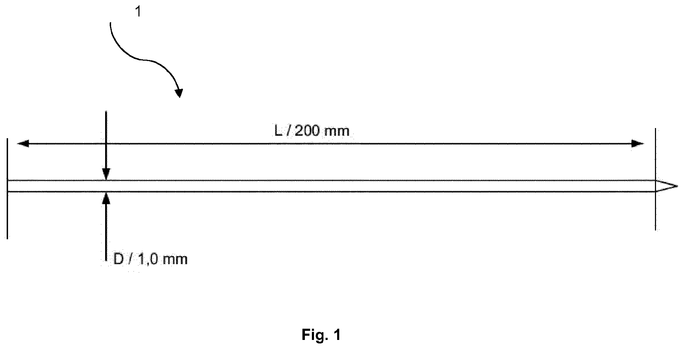

Fig. 1 zeigt eine schematische Darstellung des degradierbaren Drahtimplantats (1). Zur Herstellung des biodegradierbaren Drahtimplantats wird vorzugsweise zunächst eine Magnesiumlegierung mit folgenden Komponenten erzeugt:

| | |
|---|---|
| Seltene Erden: | 8,4 Gew.-% |
| (davon Neodym: | 2,1 Gew.-%) |
| Yttrium: | 1,6 Gew.-% |
| Zirkonium: | 0,4 Gew.-% |
| Zink: | 0,6 Gew.-% |

**Nachweisbare Verunreinigungen:**

| | |
|---|---|
| Eisen: | 0,013 Gew.-% |
| Kupfer: | 0,036 Gew.-% |
| Nickel: | 0,003 Gew.-% |
| Aluminium: | 0,0032 Gew.-% |
| Lithium: | 0,0035 Gew.-% |

Der Rest ist Magnesium.

Ein gefertigtes Magnesiumformteil wird zum gewünschten Drahtimplantat weiterverarbeitet.

Durch kaltes Drahtziehen wurde zunächst ein Draht von 1,0 mm Durchmesser (D) und 60 cm Länge (L) erzeugt. Dieser wurde in 3 gleiche Stücke von 200 mm Länge (L) geschnitten, um den Einfluss der Wärmebehandlung aufzuzeigen (1). Draht 1 wurde im originalen, gezogenen Zustand belassen. Die Werte der mechanischen Festigkeit sind in Tab. 1 Spalte 1 angegeben. Draht 2 wurde 1 h bei 490 °C +/- 10 °C unter Argon Atmosphäre lösungsgeglüht, in kaltem Wasser abgeschreckt, danach 48 h ausgelagert bei 180°C. Die Werte der mechanischen Festigkeit des so hergestellten K-Drahtes sind in Tab. 1 Spalte 2 abgebildet. Draht 3 wurde in einem Labor-Muffelofen unter Argon Schutzgas bei 400°C 60 Minuten geglüht und sehr langsam, für etwa 8 h, im Ofen auf RT abgekühlt. Die Werte der mechanischen Festigkeit sind in Tab. 1 Spalte 3 dargestellt. Dasselbe Experiment wurde mit einem Draht von 0,8 mm Durchmesser und 60 cm Länge durchgeführt. Die entsprechenden Ergebnisse sind in Tabelle 2 aufgezeigt.

Die Tabellen 1 und 2 geben Werte der Zugfestigkeit (in MPa = N/mm²), der Dehngrenze (in MPa = N/mm²) und der Bruchdehnung (in % der Länge) für degradierbare Drahtimplantate im kaltgezogenen, im ausgelagerten und im weich geglühten Zustand an. Es wurde jeweils der Mittelwert aus drei Messungen angegeben.

**Tabelle 1: Festigkeitseigenschaften erfindungsgemäß hergestellter degradierbarer Drahtimplantate nach unterschiedlicher Wärmebehandlung / Durchmesser 1 mm**

| | Draht 1 | Draht 2 | Draht 3 |
|---|---|---|---|
| 1 mm Durchmesser | Kalt gezogen | ausgelagert | weich geglüht |
| Zugfestigkeit Rm (MPa) | 360 | 402 | 281 |
| Dehngrenze Rp (MPa) | 303 | 381 | 262 |
| Bruchdehnung εB (%) | 9,7 | 2,2 | 21,3 |
| Zustand n. DIN EN 515 | F | T6 | 0 |

**Tabelle 2: Festigkeitseigenschaften erfindungsgemäß hergestellter degradierbarer Drahtimplantate nach unterschiedlicher Wärmebehandlung / Durchmesser 0,8 mm**

| | Draht 1 | Draht 2 | Draht 3 |
|---|---|---|---|
| 0,8 mm Durchmesser | Kalt gezogen | ausgelagert | weich geglüht |
| Zugfestigkeit Rm (MPa) | 372 | 432 | 287 |
| Dehngrenze Rp (MPa) | 332 | 412 | 269 |
| Bruchdehnung εB (%) | 4,3 | 2,1 | 22,4 |
| Zustand n. DIN EN 515 | F | T6 | 0 |

Durch das Weichglühen werden die Werte der Dehngrenze signifikant erniedrigt und die Bruchdehnung steigt signifikant an. Dies führt in der Praxis zu einer guten Form- und Biegbarbarkeit des Drahts an den so behandelten Stellen.

Durch das Auslagern werden insbesondere hohe Festigkeiten erzielt, welche insbesondere die Zug- und Torsionsfestigkeit des Drahtes an den so behandelten Stellen verbessern.

Die unterschiedlichen Möglichkeiten der Wärmebehandlung wie Auslagern und Weichglühen können auch an einem Draht angewendet werden. So kann beispielsweise ein Draht mit einer Spitze mit hoher Festigkeit erzeugt werden und der übrige Teil des Drahtes ist weicher uns biegsamer.

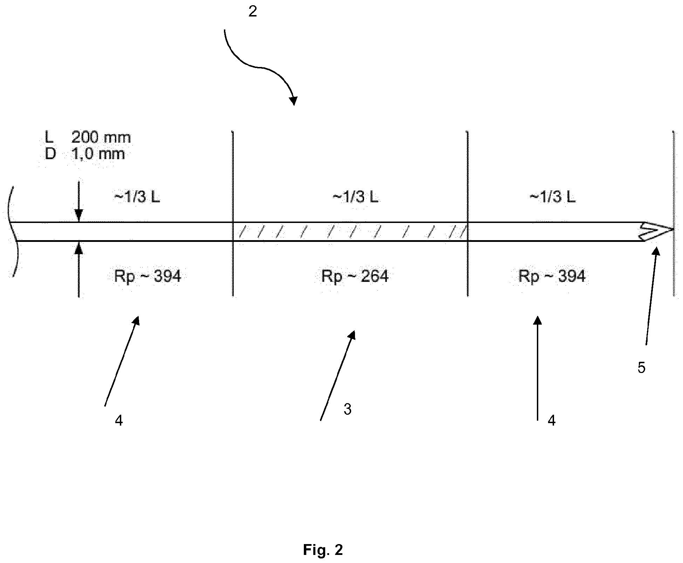

Fig. 2 zeigt ein biodegradierbares Drahtimplantat (2), welches im mittleren Drittel (3) mittels Weichglühen wärmebehandelt wurde. Durch kaltes Drahtziehen wurde zunächst ein Draht von 1,0 mm Durchmesser (D) erzeugt und auf 200 mm Länge (L) abgeschnitten.

Die Dehngrenze beträgt in diesem wärmebehandelten Abschnitt 264 MPa, in den beiden nicht-wärmebehandelten Abschnitten im vorderen und hinteren Drittel (4) 394 MPa. Die Wärmebehandlung in diesem Teilabschnitt des Implantats ermöglicht es, den biodegradierbaren K-Draht im Bereich der Trokar-Spitze (5) unverändert hart und zäh belassen werden, im mittleren Bereich form- und biegbar zu gestalten und im hinteren Bereich, dem Führungsbereich, hart und zäh zu belassen.

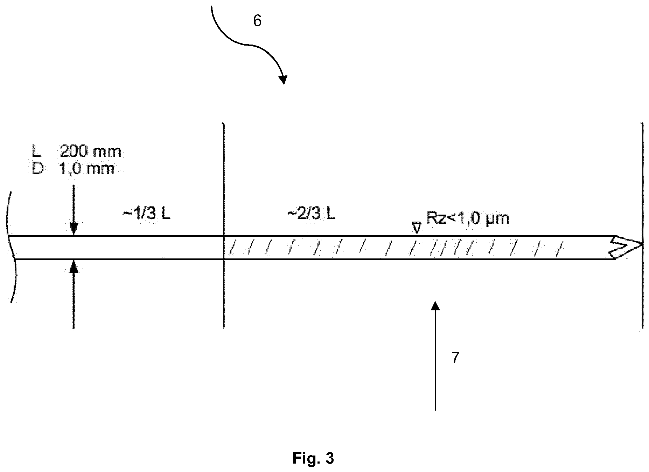

Zusätzlich zur Wärmebehandlung kann das degradierbare Drahtimplantat durch Polieren oder durch Oxidieren und Polieren der Oberfläche in ihrer Korrosionsgeschwindigkeit durchgehend oder abschnittsweise variiert und an den medizinischen Fall angepasst werden. Fig. 3 zeigt einen solchen Draht (6). Dieser wurde im mittleren Drittel wärmebehandelt durch Auslagern, und zusätzlich über die vorderen zwei Drittel (7) der Länge mittels oszillierendem Schleifen poliert, so dass eine Rauigkeit (Rz, gemittelte Rautiefe) von < 1 µm vorliegt.

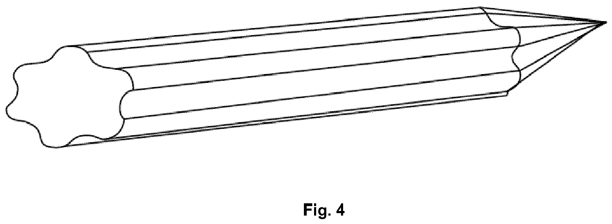

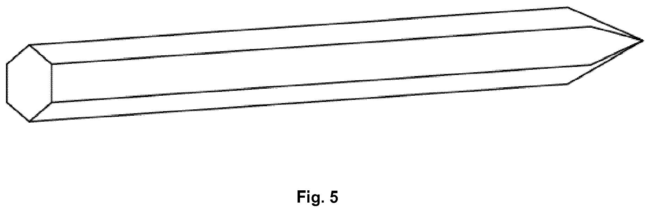

Anstelle runder Drähte können durch Strangpressen oder durch Ziehen mittels geeigneter Matrizen auch mehrkantige Drähte, beispielsweise drei-Kant oder vier-Kant, oder kanülierte Drähte mit Längsrillen erzeugt werden. Fig. 4 zeigt ein biodegradierbares Drahtimplantat mit 6 - kantigem Außenprofil, Fig. 5 ein biodegradierbares Drahtimplantat mit kanneliertem Profil.

Es sind zahlreiche Abwandlungen und Weiterbildungen der beschriebenen Ausführungsbeispiele verwirklichbar.

### Bezugszeichen

- 1.: biodegradierbares Drahtimplantat
- 2.: wärmebehandeltes, biodegradierbares Drahtimplantat
- 3.: wärmebehandelter Bereich im mittleren Drittel
- 4.: nicht-wärmebehandelten Abschnitt
- 5.: Trokar-Spitze
- 6.: Glatt poliertes, biodegradierbares Drahtimplantat
- 7.: Abschnitt mit Rauigkeit (Rz, gemittelte Rautiefe) von < 1 µm

- D: Durchmesser
- L: Länge
- Rp: Dehngrenze (MPa)
- Rz: Rautiefe (µm)

### zitierte Literatur

### zitierte Patentliteratur

WO 2015/137911 A1
WO 2017/035072 A1
EP 2 753 373 B1

## Patentansprüche

1. Drahtimplantat, insbesondere zur Spickdrahtosteosynthese,
**dadurch gekennzeichnet, dass**
das Drahtimplantat einer Wärmebehandlung unterzogen wurde,
wobei das Drahtimplantat aus einer biokompatiblen, biokorrodierbaren Magnesiumlegierung besteht, die zusammengesetzt ist aus metallischem Magnesium von mindestens 80 Gew. %, einem Anteil von Zink von 0,1 bis 2,0 Gew. %, einem Anteil von Zirkonium von 0,1 bis 2,0 Gew. %, einem Anteil von Selten-Erd-Metallen von 0,1 bis 10 Gew. % - wobei der Yttrium Gehalt unter dem Selten-Erd-Metall-Anteil 0,1 bis 5,0 Gew. % beträgt, einem Anteil von Mangan von 0,01 bis 0,2 Gew. %, einem Anteil von Aluminium von weniger als 0,1 Gew. %, einem Anteil von Kupfer, Nickel und Eisen von jeweils weniger als 0,10 Gew. %, und einem Anteil sonstiger physiologisch unerwünschte Verunreinigungen von insgesamt weniger als 0,8 Gew. %, enthält wobei der Rest der Legierung zu 100 Gew. % Magnesium ist.

2. Drahtimplantat nach dem vorhergehenden Anspruch,
**dadurch gekennzeichnet,**
**dass** die Wärmebehandlung über die gesamte Länge des Drahtes oder in mindestens einem Teilabschnitt erfolgt.

3. Drahtimplantat nach einem der vorhergehenden Ansprüche,
**dadurch gekennzeichnet,**
**dass** die Bruchdehnung in einem weichgeglühten Bereich mindestens 18% beträgt, vorzugsweise mindestens 20 %.

4. Drahtimplantat nach einem der vorhergehenden Ansprüche 1 oder 2,
**dadurch gekennzeichnet,**
**dass** die Bruchdehnung in einem dem Auslagern unterzogenen Bereich maximal 3,5% beträgt.

5. Drahtimplantat nach einem der vorhergehenden Ansprüche,
**dadurch gekennzeichnet,**
**dass** die Dehngrenze in einem dem Auslagern unterzogenen Bereich mindestens 360 MPa beträgt, vorzugsweise mindestens 380 MPa

6. Drahtimplantat nach einem der vorhergehenden Ansprüche 1 bis 4,
**dadurch gekennzeichnet,**
**dass** die Dehngrenze in einem weichgeglühten Bereich mindestens 240 MPa beträgt

7. Drahtimplantat nach einem der vorhergehenden Ansprüche,
**dadurch gekennzeichnet,**
**dass** die Zugfestigkeit in einem weichgeglühten Bereich mindestens 390 MPa beträgt.

8. Drahtimplantat nach einem der vorhergehenden Ansprüche,
**dadurch gekennzeichnet,**
**dass** der Draht rund oder mehrkantig oder mit Längsrillen gestaltet ist.

9. Drahtimplantat nach einem der vorhergehenden Ansprüche,
**dadurch gekennzeichnet,**
**dass** der Durchmesser 0,2 mm bis 6,0 mm beträgt, vorzugsweise 0,5 mm bis 4,0 mm und die Länge 30 mm bis 600 mm, vorzugsweise 50 mm bis 500 mm.

10. Drahtimplantat nach einem der vorhergehenden Ansprüche,
**dadurch gekennzeichnet,**
**dass** das Drahtimplantat in mindestens einem Teilabschnitt glatt polierte Oberfläche aufweist.

11. Drahtimplantat nach einem der vorhergehenden Ansprüche,
**dadurch gekennzeichnet,**
**dass** das Drahtimplantat in mindestens einem Teilabschnitt eine Rautiefe von weniger als 1,0 µm aufweist, vorzugsweise von 0,8 µm.

12. Drahtimplantat nach einem der vorhergehenden Ansprüche,
**dadurch gekennzeichnet,**
**dass** das Drahtimplantat in mindestens einem Teilabschnitt hohl gestaltet ist.

13. Verfahren zur Herstellung eines Drahtimplantats nach einem der vorhergehenden Ansprüche,
**dadurch gekennzeichnet,**
das Drahtimplantates über die gesamte Länge des Drahtes oder in mindestens einem Teilabschnitt Drahtimplantat einer Wärmebehandlung unterzogen wird,
wobei das Drahtimplantat aus einer biokompatiblen, biokorrodierbaren Magnesiumlegierung besteht, die zusammengesetzt ist aus metallischem Magnesium von mindestens 80 Gew. %, einem Anteil von Zink von 0,1 bis 2,0 Gew. %, einem Anteil von Zirkonium von 0,1 bis 2,0 Gew. %, einem Anteil von Selten-Erd-Metallen von 0,1 bis 10 Gew. % - wobei der Yttrium Gehalt unter dem Selten-Erd-Metall-Anteil 0,1 bis 5,0 Gew. % beträgt, einem Anteil von Mangan von 0,01 bis 0,2 Gew. %, einem Anteil von Aluminium von weniger als 0,1 Gew. %, einem Anteil von Kupfer, Nickel und Eisen von jeweils weniger als 0,10 Gew. %, und einem Anteil sonstiger physiologisch unerwünschte Verunreinigungen von insgesamt weniger als 0,8 Gew. %, enthält wobei der Rest der Legierung zu 100 Gew. % Magnesium ist.

14. Verfahren zur Herstellung eines Drahtimplantats nach einem der vorhergehenden Ansprüche,
**dadurch gekennzeichnet,**
**dass** Lösungsglühen berührungslos durch Laserlicht oder durch induktives Erwärmen oder durch Erwärmen mittels Infrarot-Bestrahlung oder in einem klassischen Muffelofen ausgeführt wird.

15. Verfahren zur Herstellung eines Drahtimplantats nach einem der vorhergehenden Ansprüche,
**dadurch gekennzeichnet,**
**dass** das Lösungsglühen bei einer Temperatur von 300°C bis 520°C erfolgt, vorzugsweise von 350°C bis 500°C, besonders bevorzugt bei 480°C.

16. Verfahren zur Herstellung eines Drahtimplantats nach einem der vorhergehenden Ansprüche,
**dadurch gekennzeichnet,**
**dass** das Lösungsglühen über einen Zeitraum von 2 bis 100 Minuten erfolgt, besonders bevorzugt 3 bis 60 Minuten.

17. Verfahren zur Herstellung eines Drahtimplantats nach einem der vorhergehenden Ansprüche,
**dadurch gekennzeichnet,**
**dass** die geringe Rauigkeit der Implantatoberfläche durch oszillierendes Kreuzschleifen, mechanisches Schleifen, Polieren, Läppen oder durch elektrochemischen Polierens erzielt wird.

18. Drahtimplantat erhältlich nach einem Verfahren gemäß einem der Ansprüche 13 bis 17.
